# EUROPÄISCHE PATENTSCHRIFT

(11) **EP 0 374 084 B1**
(45) Veröffentlichungstag und Bekanntmachung des Hinweises auf die Patenterteilung: **03.01.1996**
(21) Anmeldenummer: 89730207.1
(22) Anmeldetag: 13.10.1989
(51) Int. Cl.: A61B 17/58

(54) **Gleitlochplatte für die Osteosynthese**
Sliding hole plate for osteosynthesis
Plaque à trous coulissante pour l'ostéosynthèse

(30) Priorität: 11.11.1988 DE 3838774
(43) Veröffentlichungstag der Anmeldung: 20.06.1990
(73) Patentinhaber: Schmidt, Joachim, Dr.-Med., D-50858 Köln (DE)
(72) Erfinder: Schmidt, Joachim, Dr.-Med., D-50858 Köln (DE)
(74) Vertreter: Christiansen, Henning, Dipl.-Ing.

(56) Entgegenhaltungen:
- EP-A- 0 201 023
- CH-A- 373 516
- CH-A- 462 375
- DE-A- 2 808 971
- DE-B- 2 911 386
- US-A- 4 513 744

## Beschreibung

Die Erfindung betrifft eine Gleitlochplatte der im Oberbegriff des Anspruchs 1 angegebenen Art, wie sie aus der DE-B-29 11 386 bekannt ist.

Gleitlochplatten unterstützen die Reposition von Frakturteilen. Sie erzwingen eine Relativbewegung benachbarter Knochenteile durch die auf geneigten Gleitbahnen verschieblichen Köpfe der die Platten spannenden und am Knochen fixierenden Schrauben.

Das Prinzip der Gleitlochplatte basiert dabei auf der Längsverschieblichkeit der Frakturteile in Richtung zum Frakturspalt, wodurch eine Kompressionswirkung erzielt wird. Das Einsenken des Schraubenkopfes in das Gleitloch erzwingt eine Verschiebung der Knochenbruchstücke. Die Gleitlöcher seitlich des Frakturspaltes sind dabei so beschaffen, daß die Gleitbahnen für die Schraubenköpfe jeweils an den entfernt voneinander liegenden Enden der Langlochform aufweisenden Gleitlöcher vorgesehen sind.

Derartige Gleitlochplatten sind in einer großen Variantenvielfalt für die verschiedenartigsten Frakturen und auch für Splitterbrüche bekannt (z.B. Frank Schauwecker; "Osteosynthesepraxis"; 1981; Georg Thieme Verlag; Stuttgart/New York).

Die Größenordnung der erreichbaren Verschiebewege hängt von der Dicke der Gleitlochplatte und vom Anstiegswinkel der Gleitbahn für den Schraubenkopf ab. Beide Parameter sind aber aus medizinischen bzw. geometrisch-funktionellen Gründen begrenzt. Daher ist oft ein Nachspannen mittels eines Plattenspanngerätes unvermeidlich. Ein derartiges extern anzusetzendes Plattenspanngerät ist jedoch mit den Nachteilen eines vergrößerten Wundgebietes und der Notwendigkeit einer zusätzlichen Knochenbohrung behaftet. Ungünstig ist weiterhin die verlängerte Operationsdauer.

Hinsichtlich dieser Nachteile schafft die in DE-B-29 11 386 vorgeschlagene Verwendung eines auf die Druckplatte aufsetzbaren Spannblockes relativ großer Dicke mit einem Gleitloch Abhilfe, dessen Gleitbahn wegen der großen Dicke des Spannblockes wesentlich länger als diejenige der eigentlichen Druckplatte sein kann.

Der Erfindung liegt die Aufgabe zugrunde, bei einer Gleitlochplatte der eingangs genannten Gattung die oben geschilderten Nachteile zu beseitigen, d.h. eine Gleitlochplatte mit wesentlich erweitertem Verschiebeweg und mit einem ausreichend hohen interfragmentären Spanndruck über den gesamten Verschiebeweg zu schaffen.

Diese Aufgabe wird mit den kennzeichnenden Merkmalen des Anspruchs 1 gelöst.

Ein unteres, am Knochen anliegendes und der Knochenform angepaßtes Plattenelement wird flächig mit einem oberen Plattenelement verbunden. Die zweiteilige Ausführung der Gleitlochplatte bewirkt eine Erhöhung der gemeinsamen Dicke der Platten. Diese ermöglicht eine Verlängerung der Gleitbahn und damit des erreichbaren Verschiebeweges innerhalb eines sich durch beide Platten gemeinsam hindurch erstreckenden Gleitloches. Insbesondere läßt sich über den gesamten verlängerten Verschiebeweg ein ausreichend hoher interfragmentärer Spanndruck gewährleisten.

Die beiden Plattenelemente werden übereinandergelegt und unverrückbar - aber wieder lösbar - miteinander verbunden. Mindestens eine Gleitlochbohrung durchsetzt beide Plattenelemente. Die langlochartigen Durchbohrungen der Plattenelemente sind dabei so ausgeformt, daß beim Übereinanderlegen der Plattenelemente eine stetige oder auch eine ihre Steigung variierende Gleitbahn für die Unterseite des Schraubenkopfes entsteht. Das Langloch des oberen Plattenelementes ist dabei bevorzugt mit einer zusätzlichen Bohrung im Anschluß an das Ende des Gleitbahnüberganges vom oberen in das untere Plattenelement in der Größe des Schraubenkopfes versehen.

Der Querschnitt der Gleitbahn stimmt mit der Unterseite des Schraubenkopfes im wesentlichen über die ganze Länge der Gleitbahn überein. Auf diese Weise wird eine gute Führung der Schrauben erreicht.

Die Unterseite des unteren Plattenelementes ist der Querwölbung der zu behandelnden Knochen angepaßt. Daraus resultiert bei konstanter Dicke des unteren Plattenelementes eine gleichartige Wölbung des oberen Plattenelementes in Querrichtung. Das obere Plattenelement ist im Bereich seiner Unterseite entsprechend an die Form der Oberseite des unteren Plattenelementes angepaßt.

Zur Optimierung der interfragmentaren Kompression bei intertrochanteren Osteotomien ist das auf dem Knochen aufliegende Plattenelement als Winkelplatte ausgebildet. Auch andere z.B. gebogene Formen für spezielle Anwendungsfälle sind möglich, wobei entsprechend eine formmäßige Anpassung des darübergelegten Plattenelements erfolgt.

Um eine Erhöhung der Stabilität der erreichten Fixation und auch eine Vergrößerung des möglichen Verschiebeweges zu erreichen, kann das untere, an den Knochenfragmenten anliegende Plattenelement mit weiteren Gleitlöchern ausgestattet sein, welche sich nicht durch das darüberliegende Plattenelement fortsetzen.

Durch das Vorsehen nacheinander zu verwendender Gleitlöcher addieren sich deren Verschiebewege, wobei eine vorgegebene Einschraubreihenfolge einzuhalten ist. Die Gleitlöcher in den Plattenelementen sind in ihrer Länge so zu bemessen, daß am Ende der Gleitbahn der zuerst festzuziehenden Schrauben noch ein genügender Verschiebeweg (mit nur geringer Neigung) zur Verfügung steht, um die durch die Wirkung der weiteren Gleitlöcher resultierende relative Gleitbewegung zwischen Knochen und Platte aufzunehmen.

Das aufliegende, kraft- oder formschlüssig mit dem unteren Plattenelement verbindbare obere Plattenelement läßt sich nach dem Festziehen der Schraube(n) entlang der gemeinsamen Gleitbahn(en) entfernen. Damit werden die weiteren Gleitlöcher des an den Knochenfragmenten anliegenden Plattenelementes zugänglich.

Mit der sich durch zwei übereinanderliegende Teilelemente erstreckenden Gleitbahn eines oder mehrerer Gleitlöcher wird erstmalig die Schaffung einer Winkelplatte mit Gleitlöchern möglich, mit denen sich die bei den hier zu behandelnden Frakturen vorliegenden vergrößerten Verschiebewege mit verbesserter Verschiebbarkeit und ausreichend hohem interfragmentären Spanndruck über den gesamten Verschiebeweg bewerkstelligen lassen.

Vorteilhafte Weiterbildungen der Erfindung sind in den Unteransprüchen gekennzeichnet bzw. werden nachstehend zusammen mit der Beschreibung einer bevorzugten Ausführung der Erfindung anhand der Figuren näher dargestellt. Es zeigen:
Figur 1 einen Längsschnitt durch ein Ausführungsbeispiel der erfindungsgemäßen Gleitlochplatte in der Ausführung als Winkelplatte,
Figur 2 eine Ausführungsform eines Langloches mit der Gleitbahn in Draufsicht und
Figur 3 die beiden Teilelemente der Gleitlochplatte gemäß Figur 1 in getrenntem Zustand.

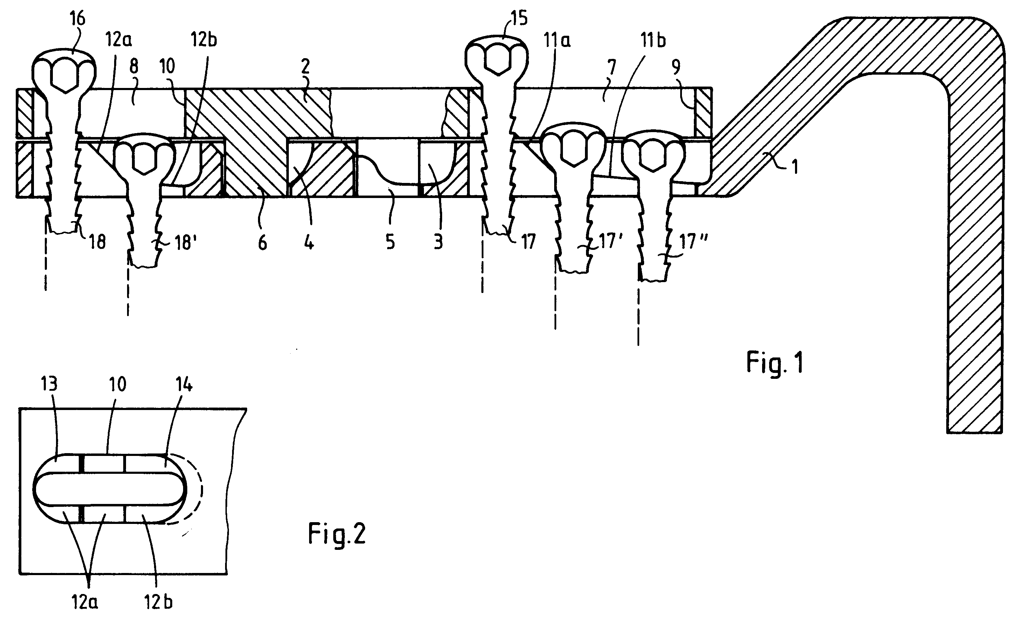

Das in Figur 1 dargestellte Ausführungsbeispiel der erfindungsgemäßen Gleitlochplatte besteht aus einem als Winkelelement 1 ausgeführten unteren Plattenelement und einem oberen Plattenelement 2.

Durch an den Plattenelementen 1 und 2 vorgesehene Verbindungselemente, welche eine Kraftübertragung in Schubrichtung von dem oberen Element 2 auf das untere Element 1 bewirken, erfolgt eine kraft- oder formschlüssige Verbindung der beiden Plattenteile. Zweckmäßigerweise ist diese Verbindung der Plattenelemente in Form einer Steckverbindung vorgesehen.

Dazu lassen sich im Winkelelement 1 vorhandene weitere Gleitlöcher 3 und 4 nutzen. Das obere Plattenelement 2 wird dazu mittels zweier, an die Form der durch die Gleitlöcher 3 und 4 gebildeten Ausnehmungen angepaßter Ansätze 5 und 6 formfest in die Gleitlöcher 3 und 4 des Winkelelementes 1 eingepaßt.

Durch die Verbindung der Plattenelemente 1 und 2 erhält das System die für den erwünschten Spannweg und den erforderlichen interfragmenteren Spanndruck notwendige Dicke. Außerdem erhöht sich auch die Stabilität.

Beide Plattenelemente sind mit zwei gemeinsamen Gleitlöchern 7 und 8 versehen. Die als Langlöcher 9 und 10 ausgeführten Gleitlöcher 7 und 8 sind mit Gleitbahnen 11a/11b und 12a/12b versehen, welche sich ausgehend von einer Ansenkung 13 an einem Ende des Langloches über dessen gesamte Längsausdehnung nach unten abgeschrägt durch beide Plattenelemente 1 und 2 erstrecken.

Die Gleitlochbahnen bestehen vorzugsweise aus zwei Abschrägungsbereichen unterschiedlicher Neigung (Bereich 11a bzw. 12a und Bereich 11b bzw. 12b). Der Bereich mit der geringeren Neigung 11b bzw. 12b befindet sich dabei innerhalb des unteren Plattenelementes 1 und entspricht in seiner Länge dabei jeweils der Gesamtlänge aller Gleitbahnen, die, der vorgegebenen Einschraubreihenfolge entsprechend, nachfolgend mit Schrauben bestückt werden. Der Gleitbahnbereich 11b ist wesentlich länger als der Gleitbahnbereich 12b, da zunächst in dem zur Gleitbahn 11a/11b gehörenden Gleitloch 7 eine Schraube eingesetzt wird und danach erst in dem zur Gleitbahn 12a/12b gehörenden Gleitloch 8. Beim Festziehen der im Gleitloch 8 eingesetzten Schraube wird auch die zuvor im Gleitloch 7 eingesetzte Schraube entlang ihres Gleitbahnbereiches 11b mitverschoben, so daß sich der Verschiebeweg, der entlang des Gleitbahnbereiches 11b erreichbar ist, als Summe der Verschiebewege entlang der Gleitbahnabschnitte 12a und 12b ergibt.

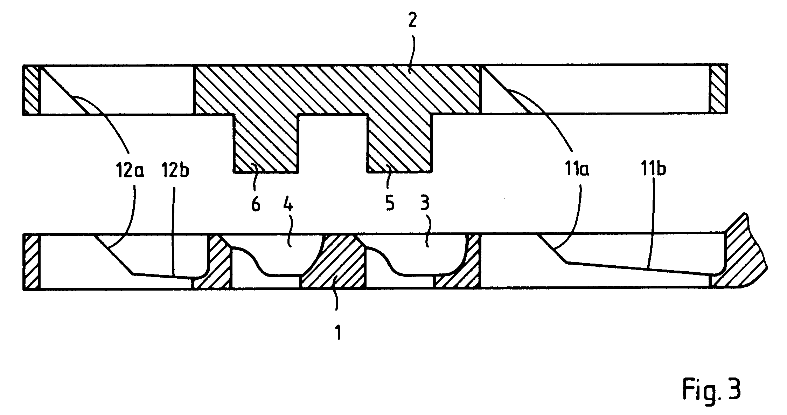

Das obere Plattenelement 2 ist innerhalb des Langloches 9 bzw. 10 mit einem zusätzlichen Durchbruch 14 für den Durchtritt des Schraubenkopfes 15 bzw. 16 versehen. Dieser Durchbruch ist in der Draufsicht auf Teile der Platten in Figur 2 erkennbar.

Das klingennahe Gleitloch 7 ist für einen Verschiebeweg von etwa 8 mm [entsprechend: 3 mm (entlang dem Teil 11a) + 3 mm + 1 mm + 1 mm (entlang dem Teil 11b)] und das klingenferne Gleitloch 8 für einen Verschiebeweg von etwa 5 mm [entsprechend: 3 mm (entlang 12a) + 1 mm + 1 mm (entlang dem Teil 12b)] ausgelegt. Eine Schraube 17 wird mit einer exzentrischen Bohrlehre am äußersten Ende des klingennahen Gleitloches 7 angesetzt. Beim Anziehen der Schraube 17 wird ein Spannweg von 3 mm entlang der Gleitbahn 11a erreicht. Die Schraube 17 befindet sich jetzt in Position 17′. Danach erfolgt das Ansetzen der Schraube 18 mit einer exzentrischen Bohrlehre am äußersten Ende des klingenfernen Gleitloches 8. Beim Anziehen der Schraube 18 wandert diese analog zu Schraube 17 ebenfalls um ca. 3 mm entlang ihres Gleitbahnbereiches 12a. Bei diesem Schraubvorgang wird die Schraube 17 um weitere 3 mm bis zur Position 17˝ mitverschoben. Insgesamt läßt sich damit ein Spannweg von 6 mm = 3 mm + 3 mm ausführen.

Die Unterseiten der Schraubenköpfe 15 und 16 sind ballig bzw. halbkugelförmig ausgebildet. Ihre Kontaktflächen mit den Gleitbahnen 11 und 12 lassen sich als Kompromiß zwischen guter Paßform und Leichtgängigkeit des Schraubvorganges optimieren. Dazu ist der Querschnitt der Gleitbahnen 11 und 12 vorzugsweise im Kantenbereich angefast.

Das Plattenelement 2 ist mit einem geeigneten Gerät durch Abziehen abnehmbar. Damit sind die beiden Gleitlöcher 3 und 4 des Winkelelementes 1 zugänglich. Das Einsetzen weiterer Schrauben in die Gleitlöcher 3 und 4 erhöht zum einen die Stabilität und ermöglicht zum anderen ein nochmaliges weiteres Annähern der Frakturteile um jeweils ca. 1mm. Beim Anziehen der Schraube innerhalb des Gleitloches 3 wandert diese um etwa 1mm in Richtung zum Frakturspalt und nimmt die Schrauben 17 und 18 mit. Die nachfolgend in das Gleitloch 4 einzusetzende Schraube bewirkt ein nochmaliges Spannen um 1mm, wobei ebenfalls alle zuvor eingesetzten Schrauben um den gleichen Verschiebeweg entlang ihrer Gleitlöcher mitgenommen werden. Insgesamt beträgt der nunmehr erreichte Spannweg ca. 8 mm = 6 mm + 1 mm + 1 mm.

Das Plattenelement 2 läßt sich problemlos sterilisieren und zur Wiederverwendung vorbereiten.

Die beiden voneinander getrennten Plattenelemente 1 und 2 sind noch einmal teilweise in Figur 3 dargestellt. Hier ist insbesondere auch die Form der Erhebungen 5 und 6 deutlich erkennbar, welche die Form der durch die Gleitlöcher 3 und 4 gebildeten Ausnehmungen in positiver Anformung nachbilden. Aus Figur 3 ist ersichtlich, daß die erfindungsgemäße Knochenplatte auch in symmetrischer Ausführung als flache Platte verwendbar ist.

Bei dem dargestellten Ausführungsbeispiel der Anwendung des erfindungsgemäßen Gleitlochsystems auf Winkelplatten läßt sich ein vergrößerter Spannweg mit Gleitlöchern ohne nachteilige Auswirkungen auf die Benutzbarkeit der Platte realisieren. Die implantierte Klinge entspricht in ihrer Größe und Stabilität den bisherigen Winkelplatten. Der konstruktionsbedingt um ca. 9 mm längere Schaft wirkt sich dabei nicht nachteilig aus.

Durch den Fortfall der Verwendung eines externen Plattenspanners mit dem Nachteil eines größeren Wundgebietes und einer zusätzlichen Knochenbohrung wird die Operation erleichtert und die Operationsdauer verkürzt.

## Patentansprüche

1. Gleitlochplatte für die Osteosynthese mit
einem ersten, auf dem Knochen aufliegenden, unteren Plattenelement (1) und
einem zweiten, auf dem unteren Plattenelement flächig aufliegenden, oberen Plattenelement (2),
wobei die Plattenelemente (1,2) je einen sich in Plattenlängsrichtung erstreckenden ovalen Durchbruch (9 bzw. 10) für den Schaft einer Schraube (17, 18) aufweisen, so daß mindestens ein gemeinsames, beide Plattenelemente durchsetzendes Gleitloch (7 bzw. 8) gebildet wird in dem eine Gleitbahn (11a/11b bzw. 12a/12b) vorgesehen ist, und die Plattenelemente (1, 2) mindestens ein sie kraft- und/oder formschlüssig bezüglich einer Kraftübertragung von der oberen (2) auf die untere Platte (1) in der durch die Neigung der Gleitbahn (11a/11b, 12a/12b) vorgegebenen Verschieberichtung miteinander verbindendes Kupplungselement (5, 6) aufweisen,
**dadurch gekennzeichnet**, daß
beide Plattenelemente (1, 2) Teile der Gleitbahn (11a/11b bzw. 12a/12b) des Gleitlochs (7 bzw. 8) aufweisen, die beim Übereinanderlegen der Plattenelemente eine stetige oder in ihrer Steigung variierende Gleitbahn bilden.

2. Gleitlochplatte nach Anspruch 1, **dadurch gekennzeichnet**, daß der im zweiten Plattenelement (2) befindliche Teil der Gleitbahn im Anschluß an das niedrigere Ende (11b bzw. 12b) der Gleitbahn einen Durchbruch (14) aufweist, dessen Querschnitt mindestens dem des Schraubenkopfes entspricht.

3. Gleitlochplatte nach einem der vorangehenden Ansprüche, **dadurch gekennzeichnet**, daß ein Teil der Gleitbahn (11b bzw. 12b) innerhalb des unteren Plattenelementes vorzugsweise wesentlich verringerte Neigung aufweist und in der Längsrichtung der Länge der Gleitbahn mindestens eines anderen Gleitlochs entspricht.

4. Gleitlochplatte nach einem der vorangehenden Ansprüche, **dadurch gekennzeichnet**, daß das Kupplungselement aus einem an der Unterseite des oberen Plattenelements (2) vorgesehenen Ansatz (5 bzw. 6) besteht, der in eine daran angepaßte Ausnehmung (3 bzw. 4) an der Oberseite des unteren Plattenelements (1) eingreift.

5. Gleitlochplatte nach einem der vorangehenden Ansprüche, **dadurch gekennzeichnet**, daß das untere Plattenelement (1) mindestens ein Gleitloch (3 bzw. 4) aufweist, dessen Gleitbahn nicht an eine Gleitbahn in dem oberen Plattenelement (2) anschließt.

6. Gleitlochplatte nach einem der Ansprüche 4 oder 5, **dadurch gekennzeichnet**, daß die Ausnehmung (3, 4) durch ein Gleitloch gebildet wird.

7. Gleitlochplatte nach einem der vorangehenden Ansprüche, **dadurch gekennzeichnet**, daß das untere Plattenelement (1) als Winkelplatte ausgebildet ist.

8. Gleitlochplatte nach einem der vorangehenden Ansprüche, **dadurch gekennzeichnet**, daß das untere und obere Plattenelement (1, 2) Teile eines Rohrquerschnitts bilden, wobei der Außendurchmesser der Krümmung der unteren Platte dem Innendurchmesser der Krümmung der oberen Platte entspricht.

## Claims

1. Plate with slide holes for osteosynthesis with a first, lower plate element (1) resting on the bone and a second, upper plate element (2) resting flat on the lower plate element, wherein the plate elements (1, 2) each have an oval opening (9, 10 respectively) extending in the longitudinal direction of the plate for the shank of a screw (17, 18), so that at least one common slide hole (7, 8 respectively) is formed which passes through both plates and in which a slide path (11a/11b, 12a/12b respectively) is provided, and the plate elements (1, 2) have at least one coupling element (5, 6) which connects them to one another in a force-locking and/or form-locking manner with regard to a transfer of force from the upper plate (2) to the lower plate (1) in the direction of displacement predetermined by the inclination of the slide path (11a/11b, 12a/12b), characterised in that both plate elements (1, 2) have parts of the slide path (11a/11b, 12a/12b respectively) of the slide hole (7, 8 respectively) which form a slide path which is constant or varies in its gradient when the plate elements are superposed.

2. Plate with slide holes according to Claim 1, characterised in that the part of the slide path located in the second plate element (2) has an opening (14) adjoining the lower end (11b, 12b respectively) of the slide path, the cross-section of this opening corresponding at least to that of the screw head.

3. Plate with slide holes according to one of the preceding claims, characterised in that a part of the slide path (11b, 12b respectively) within the lower plate element preferably has a substantially reduced inclination and in the longitudinal direction corresponds to the length of the slide path of at least one other slide hole.

4. Plate with slide holes according to one of the preceding claims, characterised in that the coupling element consists of a projection (5, 6 respectively) which is provided on the underside of the upper plate element (2) and engages in a recess (3, 4 respectively) adapted thereto on the upper face of the lower plate element (1).

5. Plate with slide holes according to one of the preceding claims, characterised in that the lower plate element (1) has at least one slide hole (3, 4 respectively) of which the slide path does not adjoin a slide path in the upper plate element (2).

6. Plate with slide holes according to one of Claims 4 or 5, characterised in that the recess (3, 4) is formed by a slide hole.

7. Plate with slide holes according to one of the preceding claims, characterised in that the lower plate element (1) is constructed as an angled plate.

8. Plate with slide holes according to one of the preceding claims, characterised in that the lower and upper plate elements (1, 2) form parts of a tube cross-section, wherein the external diameter of the curvature of the lower plate corresponds to the internal diameter of the curvature of the upper plate.

## Revendications

1. Plaque à trous à coulisse pour ostéosynthèse comprenant
un premier élément de plaque inférieur (1), qui s'appuie sur l'os,
et un deuxième élément de plaque supérieur (2) qui s'applique à plat sur l'élément de plaque inférieur,
les éléments de plaque (1, 2) présentant chacun une ouverture ovale (9 ou 10 respectivement) qui s'étend dans la direction longitudinale de la plaque, pour recevoir la tige d'une vis (17, 18), de sorte qu'il se forme ainsi au moins un trou à coulisse commun (7 ou 8 respectivement) qui traverse les deux élemnts de la plaque et dans lequel est prévue une rampe de coulisse (11a/11b ou 12a/12b respectivement), et les éléments de plaque (1, 2) présentent au moins un élément d'accouplement qui les relie l'un à l'autre par une liaison opérant par action de force et/ou par sûreté de forme, pour la transmission des forces de la plaque supérieure (2) à la plaque inférieure (1) dans la direction du coulissement fixée par l'inclinaison de la rampe de coulisse (11a/11b, 12a/12b) caractérisée en ce que
les deux éléments de plaque (1, 2) présentent des parties de la rampe de coulisse (11a/11b ou 12a/12b, respectivement) du trou à coulisse (7 ou 8 respectivement) qui forment une rampe à pente constante ou variable lorsque les éléments de la plaque sont superposés.

2. Plaque à trous à coulisse selon la revendication 1, caractérisée en ce que la partie de la rampe de coulisse qui se trouve dans le deuxième élément (2) de la plaque présente, à la suite de l'extrémité basse (11b ou 12b respectivement) de la rampe, un perçage (14) dont la section transversale corres-pond au moins à celle de la tête de la vis.

3. Plaque à trous à coulisse selon une quelconque des revendications précédentes, caractérisée en ce qu'à l'intérieur de l'élément inférieur de la plaque une partie de la rampe de coulisse (11b ou 12b respectivement) présente une pente de préférence sensiblement réduite et correspond dans la direction longitudinale à la longueur de la rampe de coulisse d'au moins un autre trou à coulisse.

4. Plaque à trous à coulisse selon une des revendications précédentes, caractérisée en ce que l'élément d'accouplement est constitué par un ergot (5 ou 6 respectivement) prévu sur la face inférieure de l'élément supérieur (2) de la plaque, et qui s'engage dans un évidement (3 ou 4 respectivement) adapté à cet ergot, formé sur la face supérieure de l'élément inférieur (1) de la plaque.

5. Plaque à trous à coulisse selon une des revendications précédentes, caractérisée en ce que l'élément inférieur (1) de la plaque présente au moins un trou à coulisse (3 ou 4 respectivement) dont la rampe ne se raccorde pas à une rampe de l'élément supérieur (2) de la plaque.

6. Plaque à trous à coulisse selon une des revendications 4 et 5, caractérisée en ce que l'évidement (3, 4) est formé par un trou à coulisse.

7. Plaque à trous à coulisse selon une des revendications précédentes, caractérisée en ce que l'élément inférieur (1) de la plaque est constitué par une plaque coudée.

8. Plaque à trous à coulisse selon une des revendications précédentes, caractérisée en ce que l'élément inférieur et l'élément supérieur (1, 2) de la plaque forment des parties d'une section de tube, le diamètre extérieur de la courbure de la plaque inférieure correspondant au diamètre intérieur de la courbure de la plaque supérieure.
